# EUROPEAN PATENT SPECIFICATION

(11) **EP 0 241 044 B1**
(45) Date of publication and mention of the grant of the patent: **16.07.1997**
(21) Application number: 87105332.8
(22) Date of filing: 10.04.1987
(51) Int. Cl.: C12N 15/81, C12N 1/18, C12N 9/40

(54) **Construction of new alpha-galactosidase producing yeast strains and the industrial application of these strains**
Konstruktion alpha-Galaktosidase produzierender Hefestämme und deren industrielle Verwendung
Construction de souches de levure qui produisent l'alpha-galactosidase, ainsi que leur application industrielle

(30) Priority: 11.04.1986 FI 861548
(43) Date of publication of application: 14.10.1987
(73) Proprietor: Alko Group Ltd., 00180 Helsinki (FI)
(72) Inventor: Liljeström, Pirkko Liisa, SF-01280 Vantaa (FI); Tubb, Roy Stephen, Deal Kent CT14 7SL (GB); Korhola, Matti Pellervo, SF-00630 Helsinki (FI)
(74) Representative: VOSSIUS & PARTNER

(56) References cited:
- EP-A- 0 066 857
- YEAST, vol. 1, 1985; S.A. PARENT et al., pp. 83-138
- P.L. SUOMINEN, "Characterisation & Applications of the Yeast MEL1 Gene", Academic Dissertation, 1988, Helsinki (FI); p. 29
- YEAST GENETICS, EBC Congress 1985; P.G. MEADEN et al., pp. 219-226
- APPLIED & ENVIRONMENTAL MICROBIOLOGY, vol. 54, no. 1, January 1988; P.L. LILJESTRÖM-SUOMINEN et al., pp. 245-249
- GENE EXPRESSION IN YEAST, Proceedings of the Aloko Yeast Symposium, 1983, Helsinki (FI); M. KORHOLA et al., pp. 231-242
- GENE, vol. 34, 1985, Elsevier; P. MEADEN et al., pp. 325-334
- MOLECULAR & CELLULAR BIOLOGY, vol. 4, no. 7, July 1984, Washington, DC (US); M.A. POST-BEITTENMILLER et al., pp. 1238-1245
- NUCLEIC ACIDS RESEARCH, vol. 13, no. 20, 1985, Oxford (GB); P.L. LILJESTRÖM et al., pp. 7257-7267
- JOURNAL OF THE INSTITUTE OF BREWING, vol. 92, November-December 1986; R.S. TUBB et al., pp. 588-590
- FEMS MICROBIOLOGY LETTERS, vol. 34, 20 March 1986, Amsterdam (NL); H. RUOHOLA et al., pp. 179-185
- JOURNAL OF THE INSTITUTE OF BREWING, vol. 89, no. 3, May-June 1983, London (GB); G.G. STEWART et al., pp. 170-188

## Description

The object of this invention are new Saccharomyces cerevisiae yeast strains transformed with an α-galactosidase gene (MEL) using recombinant DNA methods. Baker's and distiller's yeasts producing α-galactosidase are used in the corresponding industry because they are able to utilize the raffinose contained in molasses. This results in a greater yield of yeast (or ethanol) and in reduction or elimination of the costs associated with the biological oxygen demand (B.O.D.) in the effluent of factories. As a result of the improved ability of brewer's yeasts to produce α-galactosidase a sensitive method for monitoring pasteurization of beer is provided.

The new yeast strains prepared by using recombinant DNA methods produce more α-galactosidase than naturally occurring α-glactosidase producing yeast strains.

Methods for marking yeast strains and for producing stable transformants of yeasts are also provided.

α-Galactosidase (E.C. 3.2.1.22) is an enzyme able to hydrolyse melibiose to galactose and glucose. Some natural yeast species (Saccharomyces cerevisiae var. oleaceus, Saccharomyces cerevisiae var. oleaginosus, S. cerevisiae var. uvarum and Saccharomyces cerevisiae var. carlbergensis) are able to produce this enzyme. However, industrially used baker's and distiller's yeast strains (which belong to S. cerevisiae) do not produce this enzyme. This results in incomplete use of raffinose of molasses, which is widely used as a substrate for the production of baker's and distiller's yeast and sometimes used for alcohol production.

This invention comprises the construction of new baker's and distiller's yeast strains, which produce increased amounts of α-galactosidase. This invention comprises also the construction of new brewer's yeast strains able to produce increased amounts of α-galactosidase. Accordingly, an improved method to monitor the pasteurization of beer is provided. Inserting an α-galactosidase gene in a given baker's, distiller's or brewer's yeast provides a method of marking own strains or e.g. the beer produced.

### Baker's and distiller's yeast

Molasses is widely used as a substrate for the production of baker's or distiller's yeast and in some countries for the production of ethanol by yeast fermentation. A typical analysis of commercially available molasses is shown in Table 1.

**Table 1.**

| Analysis of commercially available molasses. | | |
|---|---|---|
| | Average value | Range of values |
| Dry weight | 80,9 | 72,15 - 88,34 |
| Sucrose, % of d.w. | 60,0 | 55,2 - 67,1 |
| Raffinose, % of d.w. | 1,55 | 0,00 - 4,20 |
| Invert sugar, % of d.w. | 0,41 | 0,00 - 3,41 |

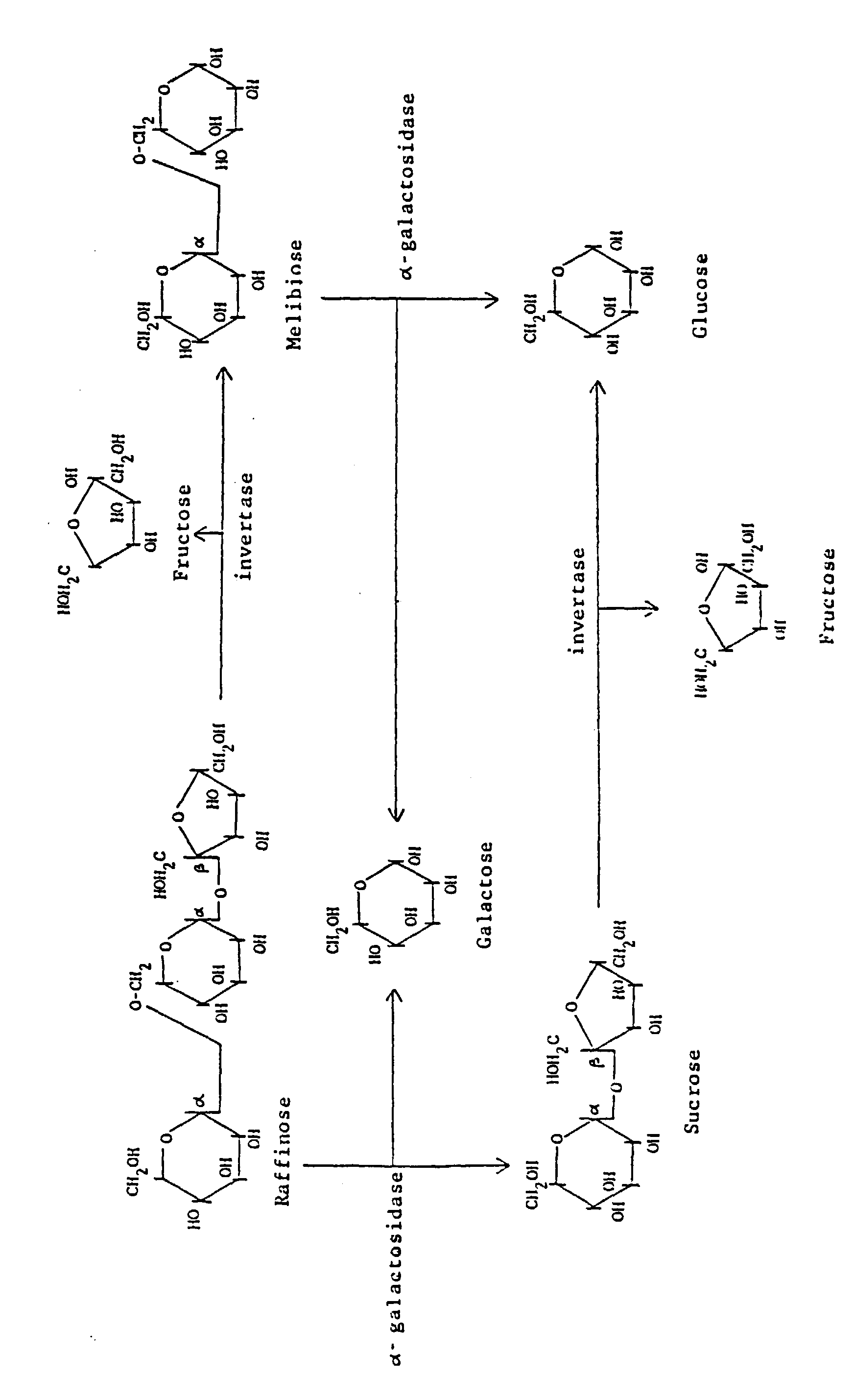

Sucrose is the major carbohydrate of molasses. It is metabolized by yeast via invertase-catalysed hydrolyses to glucose and fructose as shown in the diagram on page 3. Molasses also contain raffinose in measurable quantities (ca. 1 %). Since baking and distilling strains of yeast secrete invertase, they are able to hydrolyse raffinose to melibiose and fructose. However, most baking or distilling strains (being classified as Saccharomyces cerevisiae according to van der Walt, 1970, The Yeasts, 555-718) do not secrete α-galactosidase and therefore are unable to hydrolyse melibiose to galactose and glucose. Such strains are described as being able to utilize one third of the raffinose contained whereas strains which produce both both·invertase and α-galactosidase can utilize the raffinose completely. The construction of Mel⁺ baking or distilling strains by introducing a MEL gene conferring the capability of producing an extra-cellular α-galactosidase is therefore attractive, since the carbohydrate present in molasses can be utilized more completely. This results in:
(i) A greater yield of yeast or ethanol from the metabolization of molasses
(ii) Reduction or elimination of the costs associated with the melibiose contribution to biological oxygen demand (B.O.D.) in the effluent of factories using molasses as a fermentation substrate.

Baker's yeast is a widely distributed product. Consequently, yeast manufacturers find it very difficult to protect or retain their own strains which are, in the natural course of things, available to competitors. This has led to an uncontrolled exchange of strains and this inhibits any one of manufacturer committing substantial effort to strain improvement. According to this invention the MEL1 gene has been inserted into a LEU2 gene in a recipient baker's yeast strain. The recipient strain obtained carries a unique DNA fragment which can be recognized using the cloned MEL1 and/or LEU2 gene as a molecular probe(s). Thus, a method of "marking" a production strain is provided. Consequently, its use by the competitors can be readily detected and thereby prevented.

### Brewer's yeast

Strains for brewing ale (Saccharomyces cerevisiae) do not produce α-galactosidase, whereas strains for brewing lager (S. cerevisiae var. uvarum or carlsbergensis) have this ability (i.e. they are Mel⁺). The presence of α-galactosidase has been demonstrated in lager beers and measurement of melibiase activity has been proposed as a method of determining the number of pasteurization units a beer has received (Enevoldsen, 1981, Carlsberg. Res. Commun. 46, 37-42; Enevoldsen, 1985, J.Am. Soc. Brew. Chemists, 63, 183-184). Standardization of pasteurization treatments is important in controlling beer quality. Houever, the melibiase inactivation method is not generally applicable at present to quantify the treatment received because of low or (with ale strains) zero amounts of enzyme produced. Introduction of a cloned MEL gene into brewing strains by recombinant DNA technology, therefore offers the possibility of extending the method of Enevoldsen to beers other than lagers and, by increasing the amount of α-galactosidase produced, improving the sensitivity and reproducibility of the method. Further, since a significant proportion of α-galactosidase remains in the yeast cell wall, surplus yeast from brewery fermentations using strains with high α-galactosidase activity, will be enriched with this enzyme. Such enriched yeast could be used for hydrolysing α-galactosides in animal feed materials and therefore should have enhanced value as a feed ingredient.

The transfer of an α-galactosidase encoding gene to brewing strains makes it possible to "mark" the own brewer's yeast strains as described in connection with baker's yeast. It makes it also possible to "mark" the produced beer. According to this invention α-galactosidase can be detected straight from beer, which is a simple way to show that the α-galactosidase producing strain has been used in beer production. In this respect, the Mel⁺ characteristic of brewing lager strains is encoded by a gene which differs substantially from MEL1 (i.e. it does not form hybrids in DNA-DNA hybridizations (Tubb, R., Liljeström, P., Torkkeli, T. & Korhola, M. (1986) 13th International Conference on Yeast Genetics and Molecular Biology, Banff, Alberta, Canada 1986, Yeast 2 (Spec. Iss.) 1986 S396). Therefore even a lager beer can be marked by transfer of the MEL1 gene into a lager strain of yeast if one has an immunological method for detecting the MEL1 α-galactosidase.

### Yeast strains for effective α-galactosidase production

α-galactosidase is a useful enzyme with application in (for example): (i) hydrolysis of raffinose to support crystallization of sucrose during refining of sugar beet (ref. Obara, J. & Hashimoto, S. 1976, Sugar Technol. Rev. 4: 209-258), (ii) reducing levels of higher galactosides (stachyose, verbascose) in feed/food materials such as soya meal or soya milk-substitute (Schuler, R., Mudgett, R.E. & Mahoney, R.R. 1985. Enzyme Microb. Technol. 7: 207-211). High levels of these galactosides cause excessive flatulence when present in the diet and this is a major limitation to the exploitation of food materials derived from seed legumes.

The use of Saccharomyces strains to produce a commercial α-galactosidase has been considered previously (Vitobello, V., Branduzzi, P. & Cimini, N., US-Patent 4376167; Olivieri, R., Panselli, P., Fascetti, E. & Ciuffolotti, P., US-Patent 4431737). For use in sugar processing, enzyme preparations are required to be free from invertase. For this reason, the use of naturally occurring Mel⁺Suc⁻ strains of S. cerevisiae var. oleaceus or S. cerevisiae var. oleaginosus has been proposed.

According to this invention new yeast strains have been constructed, which produce levels of α-galactosidase which are 2 to 8-fold higher than those produced by naturally occurring strains. α-Galactosidase could be produced also as a byproduct of baker's or distiller's yeast production.

### A method for introducing recombinant DNA into industrial yeast strains and for obtaining stable transformants

Obtaining transformants of yeast strains requires a good method of selecting those yeast cells which have taken up the DNA of interest. In fundamental studies with Saccharomyces cerevisiae, this is usually accomplished by including within the recombinant DNA a selectable marker gene (e.g. LEU2, HIS3, URA3, TRP1) which complements a corresponding auxotrophic mutation in the chosen recipient. After treatment with DNA, protoplasts or cells are plated on (or in) a medium which does not provide the auxotrophic requirement of the recipient strain. Therefore, only transformants are able to grow.

Brewing, baking, distilling or vine yeasts are usually wild type strains (i.e. they do not have any auxotrophic requirements) and it is neither practicable nor desirable to introduce auxotrophic mutations into these strains. Therefore, transformation of industrial yeasts requires the use of dominant marker genes which are selectable against a wild-type polyploid background. For example, genes conferring resistance to the aminoglycoside antibiotic G418 (Webster, T.D. & Dickson, R.C. (1983). Gene 26: 243-252) or to copper ions (Henderson, R.C.A., Cox, B.S. and Tubb, R. (1985) Current Genetics 9: 133-138) have been used. Another possibility is the use of a gene conferring the ability to grow on a novel substrate. We show that the yeast MEL1 gene can be introduced into industrial yeast strains by selecting transformants on a medium containing melibiose as sole carbon source. With a medium containing the chromogenic substrate x-α-galactoside (5-bromo-4-chloro-3-indolyl-α-D-galactoside) putative Mel⁺ transformants producing α-galactosidase can be detected. X-α-galactoside can be incorporated into the medium initially used for obtaining transformants and/or used during subsequent assays. Mel⁺ colonies turn blue-green on media containing the x-α-galactoside. This is a convenient way of identifying those transformants which retain the transferred DNA in a stable way. Unstable transformants segregate white colonies on media containing the x-α-galactoside. Therefore yeast strains transformed with a MEL gene can be readily selected and the presence of the gene can be readily scored using the x-α-galactoside. Thus, MEL gene is a marker gene of wide applicability in recombinant DNA technology.

The x-α-galactoside substrate can be used also for the detection of α-galactosidase in beer and for differentiating ale Mel⁻ from lager Mel⁺ strains of yeast (R. Tubb and P. Liljeström, "A colony-colour method which differentiates α-galactosidase-positive strains of yeast", J.Inst.Brew. 92 (1986), pp. 588-590).

The new yeast strains constructed according to this invention are M12E-2, M20E-1 and N12E-1. They have been deposited on 27 March, 1986 with the National Collection of Yeast Cultures. The deposit numbers are NCYC 1567, NCYC 1568 and NCYC 1569, respectively.

This invention is illustrated by the following drawings and examples.

### BRIEF DESCRIPTION OF THE DRAWINGS

- Figure 1.: Construction of the plasmid pALK12
- Figure 2.: Southern blot of MK270 and M12E-2 by using as a probe A) pBR 322-DNA, B) MEL1-DNA and C) LEU2-DNA. Lane 1 is DNA of MK270 and lane 2 is DNA of M12E-2.
- Figure 3.: Construction of the plasmid pALK20
- Figure 4.: Construction of the plasmid pALK52.
- Figure 5.: Accumulation of melibiose during baker's yeast growth measured by HPLC after A) 10 h B) 16 h growth period.
- Figure 6.: A greater yield of yeast due to additional raffinose
- Figure 7A.: Total α-galactosidase production of different strains during growth
- Figure 7B.: α-Galactosidase activity secreted into the growth medium from the same samples as in Fig. 7A.
- Figure 8.: Construction of the plasmids pALK22 and pALK23

Summing up, the present invention relates to the construction of new α-galactosidase producing yeast strains and to the industrial application of these strains. Preferred embodiments of the invention are:
- The construction of Mel⁺ (α-galactosidase-producing) baker's or distillers' yeast strains which can utilize the raffinose present in molasses, a widely used substrate for commercial yeast production and for alcohol production.
- The construction of brewer's yeast strains with enhanced capacity for producing α-galactosidase. This provides a sensitive method for monitoring pasteurization of beer and increases the value of surplus brewer's yeast as a feed supplement.
- The construction of yeast strains yielding high levels of α-galactosidase for use in enzyme production. Gene transfer to Suc-(invertase negative) strains permits the production of enzyme preparations which are not contaminated with invertase activity.

Further preferred embodiments are:
- A method for "marking" a commercial baker's yeast or brewer's yeast so that it can be unambiguously identified and which is thus protected against use by competitors.
- A method for introducing recombinant DNA into industrial yeast strains and for obtaining stable transformants.

Thus, the present invention relates to a method for the production of recombinant baker's, distiller's or brewer's yeast strains of the species Saccharomyces cerevisiae, stably comprising a gene coding for an α-galactosidase enzyme and producing increased amounts of said α-galactosidase enzyme compared to the non-transformed starting strain; said method comprising the steps of:
(a) isolating a gene coding for an α-galactosidase enzyme from a yeast strain naturally containing a gene encoding this enzyme;
(b) linking the gene operatively to a vector able to transform and to be maintained in baker's, distiller's or brewer's yeast cells;
(c) transferring this vector or part of it to an industrial baker's, distiller's or brewer's yeast cell; and
(d) directly selecting stable yeast clones expressing said α-galactosidase as a dominant marker and making pure cultures of these, said dominant marker integrated into a chromosome being detected by using:
   (i) melibiose, or
   (ii) X-α-gal (5-bromo-4-chloro-3-indolyl-α-D-galactoside) as a substrate in the selection and/or screening medium, or
   (iii) by using melibiose as the sole carbon source and X-α-gal as a substrate in the selection and/or screening medium, or
   selecting stable yeast clones expressing said α-galactosidase as a dominant marker and making pure cultures of these, said dominant marker contained in an autonomously replicating plasmid being detected by using:
   (i) X-α-gal as a substrate in the selection and/or screening medium, or
   (ii) by using melibiose as the sole carbon source and X-α-gal as a substrate in the selection and/or screening medium.

In a particularly preferred embodiment the MEL1 gene from Saccharomyces cerevisiae var. uvarum is used in said method.

In a further particularly preferred embodiment of said method the plasmid containg a gene coding for an α-galactosidase enzyme comprises at least part of the plasmids pALK2, pALK4, pALK12, pALK20, pALK22, pALK23 or pALK52 or any combination of these plasmids.

In a further particularly preferred embodiment of said method the transformed yeast strain does not produce invertase.

In a further particularly preferred embodiment of said method the transformed yeast strain is MK270.

In a further particularly preferred embodiment of said method the transformed yeast strain is NCYC 1245.

In a further particularly preferred embodiment of said method the yeast strain is YF135.

In a further particularly preferred embodiment of said method the yeast strain is M12E-2, M20E-1, N12E-1, pALK22/YF135, pALK23/YF135, pALK4/MK270 or pALK52/MK270.

In a further particularly preferred embodiment of said method the stable yeast strains are selected by using melibiose as the sole carbon source.

In a further particularly preferred embodiment of said method the stable yeast strains are selected by using X-α-gal as a substrate in the selection or screening medium.

A further preferred embodiment of the present invention is a baker's, distiller's or brewer's yeast strain of the species Saccharomyces cerevisiae, characterized in that it is an industrially applicable transformer of a mild-type strain stably comprising a gene coding for an α-galactosidase enzyme, said yeast strain being able to produce increased amounts of α-galactosidase enzyme. A particularly preferred embodiment of said yeast strains is a yeast strain wherein the gene coding for an α-galactosidase enzyme is the MEL1 gene from Saccharomyces cerevisiae var. uvarum.

A further particularly preferred embodiment of said yeast strains is a yeast strain which contains at least part of the plasmids pALK2, pALK4, pALK12, pALK20, pALK22, pALK23 or pALK52 or any combination of these plasmids. A further particularly preferred embodiment of said yeast strains is a yeast strain which does not produce invertase.

A further particularly preferred embodiment of said yeast strains is the transformed yeast strain MK270.

A further particularly preferred embodiment of said yeast strains is the transformed yeast strain NCYC 1245.

A further particularly preferred embodiment of said yeast strains is the transformed yeast strain YF135.

A further particularly preferred embodiment of said yeast strains is the yeast strain M12E-2, M20E-1, N12E-1, pALK22/YF135, pALK23/YF135, pALK4/MK270, or pALK52/MK270.

A further preferred embodiment of the present invention is a method for the production of recombinant baker's, distiller's or brewer's yeast strains of the species Saccharomyces cerevisiae, stably comprising a gene coding for an α-galactosidase enzyme and producing increased amounts of that enzyme. In that method a plasmid is used which is able to stably transform and express a gene coding for an α-galactosidase enzyme in said strains, in which the gene is the MEL1 gene from Saccharomyces cerevisiae var. uvarum.
In a particularly preferred embodiment of this method a plasmid which comprises at least part of the plasmids pALK2 (Ruokola et al., FEMS Microbiology Letters 34 (1986), 179-186), pALK4 (Ruokola et al., supra), pALK12, pALK20, pALK22, pALK23 or pALK52 or any combination of these plasmids is used.

A further preferred embodiment of the present invention is a method for the improved utilization of raffinose or higher α-galactosides from a raw material, in which a baker's or distiller's yeast is used.

In a particularly preferred embodiment this method comprises:
(a) using a baker's or distiller's yeast strain containing a gene coding for an α-galactosidase enzyme and
(b) cultivating this yeast strain using raffinose or higher α-galactosides containing raw material.
In a particularly preferred embodiment the MEL1 gene frcm Saccharomyces cerevisiae var. uvarum is used in said method.
In a further preferred embodiment of said method the baker's or distiller's yeast strain comprises at least part of the plasmids pALK2, pALK4, pALK12, pALK20, pALK22, pALK23 or pALK52 or any combination of these plasmids. In a further preferred embodiment of said method the transformed yeast strain is MK270.
In a further preferred embodiment of said method the baker's or distiller's yeast strain is M12E-2, M20E-1, pALK4/MK270 or pALK52/MK270,

A further preferred embodiment of the present invention is a method for producing an α-galactosidase enzyme in which a baker's or distillers yeast strain from the species Saccharomyces cerevisiae is used. This method comprises:
(a) transferring a gene encoding an α-galactosidase enzyme to a yeast strain
(b) growing said yeast strain under suitable conditions; and
(c) recovering and purifying the α-galactosidase enzyme.

In a particularly preferred embodiment the MEL1 gene from the species Saccharomyces cerevisiae var. uvarum is used in said method.
In a further particularly preferred embodiment of said method the yeast strain comprises at least part of the plasmids pALK2, pALK4, pALK12, pALK20, pALK22, pALK23 or pALK52 or any combination of these plasmids.
In a further particularly preferred embodiment of said method the transformed yeast strain does not produce invertase.
In a further particularly preferred embodiment of said method the transformed yeast strain is MK270,
In a further particularly preferred embodiment of said method the yeast strain is M12E-2, M20E-1, pALK22/YF135, pALK23/135, pALK4/MK270 or pALK52/MK270.

A further preferred embodiment of the present invention is a method for marking an industrially applicably yeast strain, in which a gene encoding an α-galactosidase enzyme is used as a dominant marker, said method comprising:
(a) transferring a gene encoding an α-galactosidase enzyme and integrating it into the chromosome of said strain; and
(b) detecting the gene by conventional hybridization methods.

In a particularly preferred embodiment the MEL1 gene from the species Saccharomyces cerevisiae var. uvarum is used in this method.

A further preferred embodiment of the present invention is a method for detecting the pasteurization units of beer, which comprises:
(a) transferring a gene encoding an α-galactosidase enzyme to a brewer's yeast strain which remains an autonomously replicating plasmid in said strain or which is integrated into the chromosome of said strain by homologous recombination;
(b) preparing beer using this α-galactosidase producing brewer's yeast strain, the α-galactosidase production of which is enchanced compared to the α-galactosidase production of naturally occurring brewer's yeast strains which are able to produce this enzyme;
(c) pasteurizing the beer produced; and
(d) measuring the α-galactosidase activity of the beer.

In a particularly preferred embodiment of this method the MEL1 gene from Saccharomyces cerevisiae var. uvarum is used.
In a further particularly preferred embodiment of said method the yeast strain comprises at least part of the plasmids: pALK2, pALK4, pALK12, pALK20, pALK22, pALK23 or pALK52 or any combination of these plasmids.
In a further particularly preferred embodiment of said method the α-galactosidase activity of the beer is measured by using X-α-gal as a chromogenic substrate.

The examples illustrate the invention.

### EXAMPLE 1

### Construction of a baker's yeast strain containing an α-galactosidase gene (M12E-2)

Genomic library of Saccharomyces cerevisiae var. uvarum strain ATCC9080 carrying the MEL1 gene was constructed in E. coli DH-1. (F-, end A1, hsdR17, SupE44, thi-1, recA1, gyrA96, relA1) as described by Ruohola, H. Liljeström, P., Torkkeli, T., Kopu, H., Lehtinen P., Kalkkinen, N. and Korhola, M. (1986) FEMS Microbiology Letters 34:179-185. Total genomic DNA was partially digested with Sau3A and ligated into the BamHI cut shuttle vector YEp13 (Broach, J., Strathern, J. and Hicks, J. (1979), Gene 8: 121-133). The resulting yeast DNA library consisted of ca. 11.000 Amp^{r} colonies and the average size of yeast DNA insert was 7.8 kb.

The MEL1 gene was isolated from this library by complementation of mel1-18 mutation in P18-1a. (MATa mel1-18 leu 2-3, 112 trpl ura 1) (Post-Beittenmiller, M., Hamilton, R. and Hopper, J. (1984) Mol. Cell. Biol. 4: 1238-1245) obtained from J. Hopper. The original clone which conferred the ability to produce α-galactosidase activity contained a 6.5 kb insert (pALK2). By complementation analysis the MEL1 gene was localized on a 2.8 kb BamHI/SalI fragment. The complete DNA sequence of this gene has been published (Liljeström, P., (1985) Nucl. Acids Res. 13: 7257-7268, Sumner-Smith, M., Bozzato, R., Skipper, N., Davies, R. and Hopper, J. (1985) Gene 36: 333-340, and European patent Application 0208 706). The multicopy plasmid (pALK2) carrying MEL1 gene on a 6.5 kb fragment of yeast DNA in YEp13 was transformed (Hinnen, A., Hicks, J. and Fink, G. (1978) Proc. Natl. Acad. Sci. (USA) 75: 1929-1933) into an industrially used baker's yeast strain MK270 (MK270 ferments the following sugars: galactose, glucose, fructose, mannose, maltose, sucrose and raffinose, but not the following:
lactose, melibiose and inuline. The strain assimilates the following sugars: trehalose, α-methyl-D-glucoside, glycerol, furanose and meletsitose, but not the following: inuline, sellobiose and D-gluconate).

The MEL1 gene was used as a dominant selectable marker and transformants were selected on minimal melibiose plates. Transformation frequency was 60-80 transformants/µg of DNA. When these transformants were grown on nonselective melibiose fermentation indicator plates, the Mel⁺ phenotype was found to be unstable. Only 20-30 % of the cells in a Mel⁺ colony were Mel⁺, i.e. harboring the plasmid pALK2. Mel⁺ transformants can be verified by the production of a non-diffusible blue dye on agar medium containing X-α-Gal. This same test was also applied to determining whether or not transformants were stable.

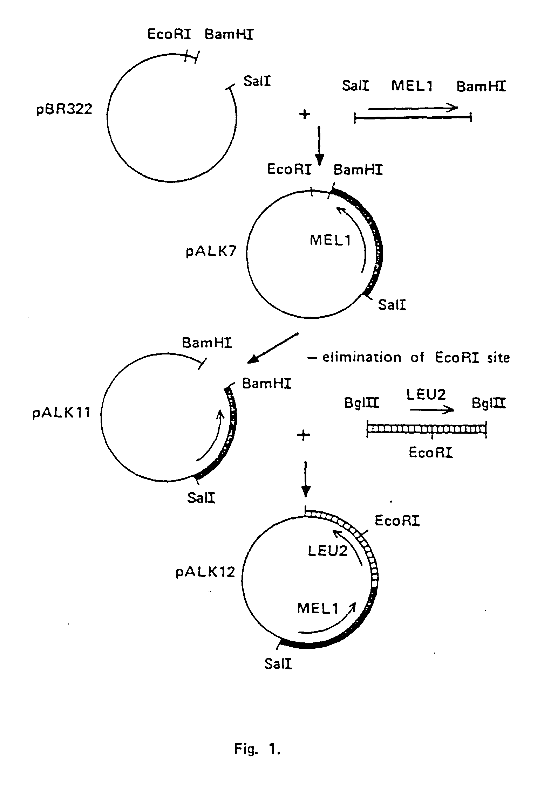

To obtain more stable transformants, pALK12 was constructed (Fig. 1) and integrated into chromosomal DNA of MK270. The MEL1 gene containing 2.8 kb SalI-BamHI fragment was isolated from pALK2 and ligated between the SalI and BamHI sites of pBR322. The resulting plasmid, pALK7 contained a unique recognition site for restriction endonuclease EcoRI. This site was destroyed by cutting the plasmid with EcoRI, filling in the sticky ends and religating. This plasmid was called pALK11. The LEU2 gene containing 2.S kb BglII-BglII fragment was isolated from YEpl3 and ligated to the unique BamHI site of pALK11.

The plasmid pALK12 carries the yeast LEU2 gene, which was used as a homologous sequence for recombination between the plasmid pALK12 and MK270 genome. The integration of pALK12 was directed to the LEU2 locus by linearization of the plasmid with EcoRI, which cuts in the middle of the LEU2 gene. Transformants were again selected on minimal melibiose plates. Stability of the Mel⁺ phenotype of these transformants was examined and 4 out of 12 were found to show 100 % stability (i.e. no Mel⁻ colonies were observed) under the test conditions. One of these, named M12E-2 was examined further.

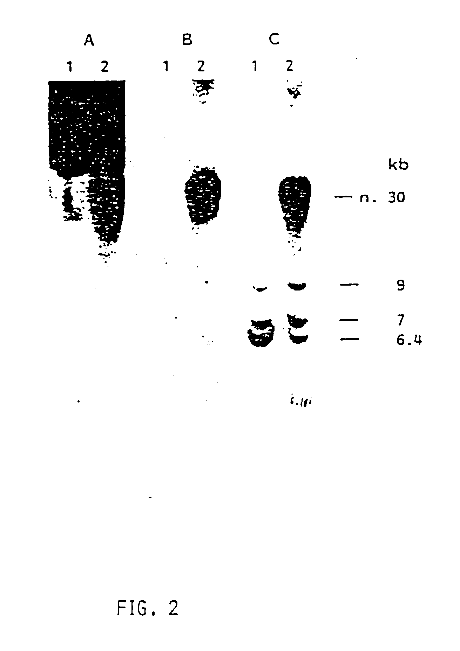

Integration of the plasmid pALK12 in M12E-2 was confirmed by a Southern blot experiment (Fig. 2). Chromosomal DNA isolated from MK270 and M12E-2 was digested with BglII, a restriction enzyme which doesn't cut within pALK12 sequences. Southern blot hybridization was performed as described in Maniatis, T., Fritsch, E. and Sambrook, J. (1982). Molecular cloning: A laboratory manual. Cold Spring Harbour Laboratory, USA.

No homology to MEL1-specific or pBR322-specific probes was found in the original bakers' yeast MK270. But as expected a new band hybridizing to MEL1-specific, pPR322 specific and LEU2-specific probes was found in M12E-2. The LEU2-specific probe recognized three bands in MK270, which is probably due to polymorphism in the BglII recognition sites in the polyploid MK270. The same bands were still present in M12E-2 indicating that the integration event had not taken place into the LEU2 locus or more likely that integration had not occurred into all homologues of chromosome III in the polyploid MK270. It is highly likely that the integration has occurred at a LEU2 locus because it has been shown that cutting the plasmid with a restriction endonuclease which recognizes a single site within a yeast gene on that plasmid (LEU2 on pALK12) increases the transformation efficiency as well as directs the integration into that gene.

### EXAMPLE 2

### Construction of a baker's yeast strain containing a gene encoding α-galactosidase without incorporating bacterial plasmid DNA (M20E-1)

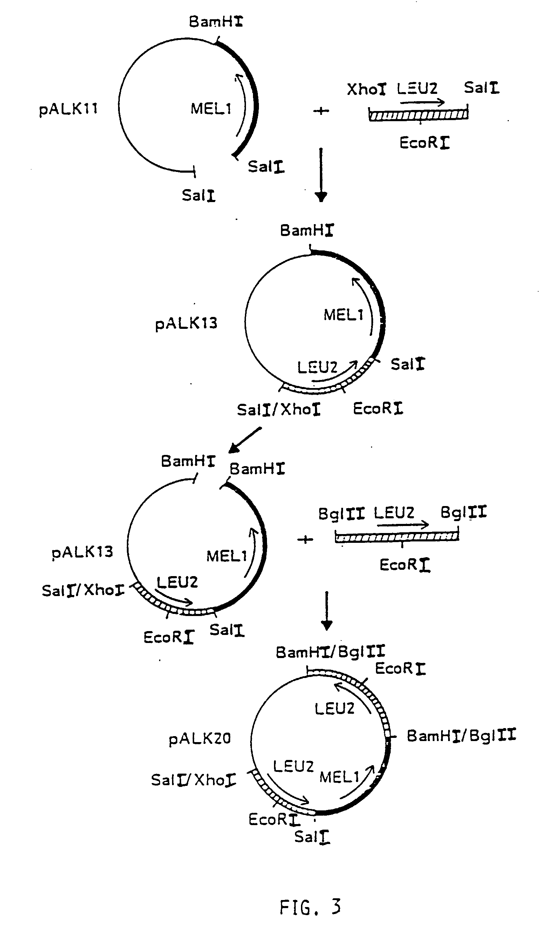

DNA integrated to the yeast chromosome in M12E-2 transformant contains sequences from bacterial plasmid pBR322. It is desirable to construct baker's yeast strains which don't contain DNA of bacterial origin. To join MEL gene into a yeast chromosome without pBR322 sequences, a new plasmid pALK20 (Fig. 3) was constructed. The LEU2 gene containing 2.3 kb XhoI-SalI fragment was isolated from YEp13 and ligated to the unique SalI site of pALK11. The resulting plasmid pALKL3 was cut with BamHI and the LEU2 gene from YEp13 as a 2.8 kb BglII fragment was ligated to the BamHI site.

Integration of MEL1 gene to the yeast (MK270) chromosome was carried out by using EcoRI fragment from pALK20, which consists solely of yeast DNA (yeast LEU2 and MEL1 genes). Again stable Mel⁺ transformants were obtained and one of these, named M20E-1 was examined further.

### EXAMPLE 3

### Construction of a baker's yeast strain containing a gene encoding α-galactosidase in an autonomously replicating Plasmid (pALK52/MK270)

A new yeast strain containing α-galactosidase coding gene but not any pBR sequences was constructed also by ligating the MEL1 gene to an autonomously replicating plasmid.

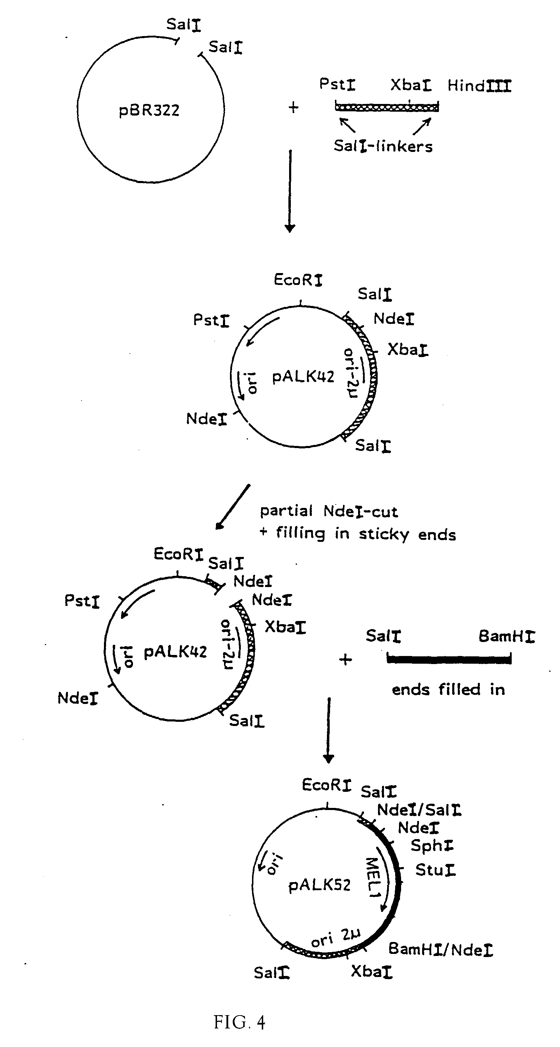

The plasmid pALK52 (Fig. 4) was constructed in the following way. 2µ origin of replication containing HindIII-PstI fragment was isolated from YEp13. Using SalI-linkers this fragment was ligated into the SalI-site of pBR322. The resulting plasmid was called pALK42. The 2.8 kb SalI-BamHI fragment containing the MEL1-gene was then ligated into the NdeI-site within the 2µ fragment in plasmid pALK42. This plasmid was called pALK52. The 4.7 kb fragment of pALK52 containing the MEL1-gene and the 2µ origin of replication was isolated and ligated into a circle, which was transformed into MK270 using the MEL1-gene as selectable marker. One of the transformants pALK52/MK270, showed a stability of more than 99.9 %/generation.

### EXAMPLE 4

### Ability of Mel⁺ baker's yeast strains (M12E-2 and M20E-1) to utilize raffinose of molasses

Mel⁺ derivatives (M12E-2 and M20E-1) of baker's yeast (MK270) were tested for their ability to utilize raffinose in molasses. The conditions used were chosen to mimic commercial baker's yeast production.

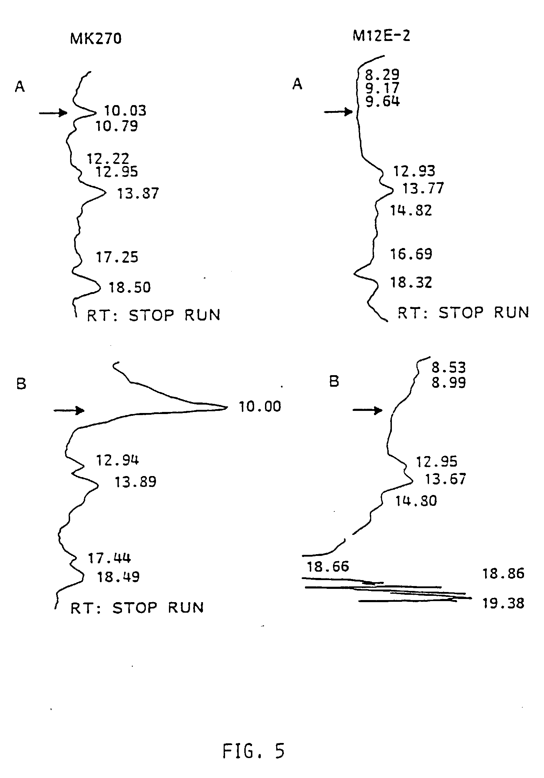

Starter cultures of strains MK270, M12E-2 and M20E-1 were grown anaerobically in minimal galactose or minimal molasses medium. 10g of yeast from these cultures was then inoculated in 1.8 l of water. Molases medium contained per litre: 14.55g of (NH₄)₂SO₄, 2.43g of NH₄H₂PO₄, 0.61g of MgCl·6H₂O, 0.12 mg of biotine and molasses corresponding to 5 % sugar. Molasses medium was fed in gradually such that about 700 ml was used during a 16h growth period. As is shown in Fig. 5, melibiose accumulates when strain MK270 is grown on molasses, indicating that the α-galactosidic bond in raffinose is not hydrolyzed. The amount of melibiose formed (0.07%) indicates that the raffinose present is totally converted to fructose and melibiose. Therefore absence of melibiose formation during growth on molasses would indicate that raffinose is completely degraded to utilizable sugars (fructose, glucose and galactose). It can be seen that when M12E-2 was used no melibiose could be detected (Fig. 5). The same was true for M20E-1 (data not shown). The α-galactosidase activity produced by M12E-2 grown on molasses is given in Table 2. In this case, the inoculum was grown on galactose which is known to be an inducer of the synthesis of α-glactosidase (Kew, O. and Douglas, H. (1976) J. Bacteriol. 125: 33-41). However, total α-galactosidase activity increases during growth on molasses, indicating the continued production of α-galactosidase. When molasses was used for growth of the inoculum, again no melibiose accumulated indicating that sufficient α-galactosidase to fully hydrolyze raffinose is also produced under these conditions.

**Table 2.**

| α-galactosidase production in molasses | | | |
|---|---|---|---|
| Strain | Time of growth (h) | Total activity (U_{B}) | Activity in medium (U_{B}) |
| M12E-2 | 10 | 2200 | 430 |
| | 16 | 3380 | 530 |
| | | | |
| MK270 | 10 | 0 | 0 |
| | 16 | 0 | 0 |

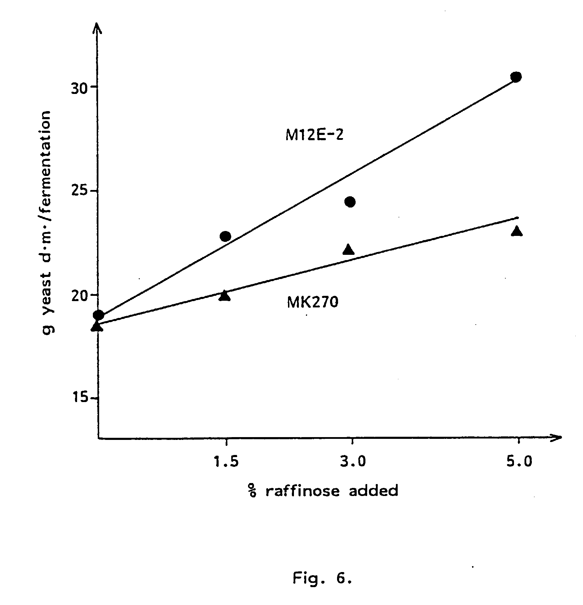

We have shown that melibiose from raffinose is used for yeast growth in the following way. Different amounts of raffinose was added to the above described fermentation medium and the fermentation was carried out as before. As is shown in Fig. 6 considerably more biomass was produced by M12E-2 when compared with the biomass yield of MK270 in the same conditions. As expected also the biomass yield of MK270 is increased with increasing amounts of raffinose. This is due to utilization of fructose from raffinose. M12E-2 can utilize also melibiose from raffinose and thus the biomass yield is even higher.

### EXAMPLE 5

### Distiller's yeast strains expressing an α-galactosidase gene

Since baker's yeast strains are sometimes also used in distilling, strains such as M12E-2 and M20E-1 could also be used in fermentations of molasses to provide greater utilization of carbohydrate and therefore, an increase in ethanol yield. Alternatively the MEL gene could be introduced, by the means described into specialized distillery yeasts such as the commercially available strain DCL"M", which has been shown to be amenable to genetic manipulation using recombinant DNA (Bussey, H. and Meaden, P. (1985) Current Genetics 9: 285-291). The ability of M12E-2 and M20E-1 to produce α-galactosidase is also compared under standard conditions with other strains in Table 3 (Example 6).

### EXAMPLE 6

### Construction of brewer's yeast strains expressing an α-galactosidase gene

In a further experiment, the MEL1 gene has been introduced into a brewing ale strain NCYC 1245 using pALK2 (nonintegrating) and by integrative transformation (pALK12). A stable Mel⁺ transformant (N12E-1) obtained by integrative transformation is shown in Table 3 to have a much greater ability to produce α-galactosidase than a brewing lager strain (NCYC 1324) (which is naturally Mel⁺). The comparatively low levels produced by brewing lager strains can also be deduced from the published data of Enevoldsen (Enevoldsen (1981) Carlberg. Res. Commun. 46: 37-42; Enevoldsen (1985). J. Am. Soc. Brew. Chemists 63: 183-184). It can be seen therefore, that although brewing lager strains are already Mel⁺, it would be useful to consider enhancing also their ability to produce α-galactosidase by use of a cloned MEL gene. In this case, however, transfer of a MEL gene by direct selection of Mel⁺ transformants as described here is not possible. An alternative strategy is to use the yeast CUP1 (conferring copper resistance) as a means to obtain transformants. The use of the CUP1 gene as a selection system for introduction of a DEX gene into a brewing lager strain has been described by Meaden, P. and Tubb, R. (1985). In: Proceedings of the 20th European Brewery Convention Congress, Helsinki pp. 219-226. IRL Press, London.

**Table 3.**

| Total induced α-galactosidase activities in different strains | | | | |
|---|---|---|---|---|
| Strain | α-galactosidase activity (U_{A}) % of total | | | % of total activity in M12E-2 |
| | Total | Cellbound | Medium | |
| ATCC 9080 | 184 | 60 % | 40 % | 14,4 % |
| M12E-2 | 1279 | 74 % | 26 % | 100 % |
| M20E-1 | 113 | 72 % | 28 % | 8,8 % |
| pALK52/MK270 | 970 | not done | not done | 76 % |
| pALK4/MK270 | 920 | not done | not done | 72 % |
| NRRLY12056 | 597 | 86 % | 14 % | 46,7 % |
| NRRLY12057 | 684 | 72 % | 28 % | 53,5 % |
| N12E-1 | 169 | 55 % | 45 % | 13,2 % |
| NCYC1324 | 8 | 100 % | not detectable | 0,6 % |
| 1453-3A | 549 | 67 % | 33 % | 42,9 % |

### EXAMPLE 7

### A new method for "marking" yeast or bacterium strains

The strains constructed in Example 1 can be easily recognized by a Southern blot. This is demonstrated in Fig. 2 using the strain M12E-2. A new fragment of DNA, which is recognizable with a MEL1, LEU2 and/or pBR322 probe is seen in M12E-2 DNA but not in MK270 DNA. The presence of this fragment distinguishes M12E-2 from any natural S. cerevisiae strain. Similarly, all other strains constructed in Example 1 are marked with a novel DNA fragment recognizable with MEL1 and/or LEU2 probes. In similar way any yeast strain could be marked using the MEL1 gene. This includes strains that already carry a MEL gene, if the MEL1 gene is directed to a new location on a chromosome (e.g. to LEU2 locus as in Example 1). Integration to a new location leads to a new restriction fragment recognizable in Southern blot.

The MEL1 gene can also be used to mark strains of bacteria, when a piece of bacterial DNA is provided on the plasmid to allow homologous recombination between recipient chromosome and the plasmid to be integrated.

### EXAMPLE 8

### α-galactosidase production of new yeast strains constructed by using recombinant DNA methods

The α-galactosidase encoded by the MEL1 gene hydrolyses melibiose, raffinose and stachyose and therefore most probably higher galactosides e.g. verbascose. Enzyme activities produced by M12E-2 against these different substrates are compared (Table 4). Activity against stachyose is needed when reduction of levels of higher galactosides in feed/food materials is considered.

**Table 4.**

| Hydrolyzed sugar mg/h/activity unit* | |
|---|---|
| Melibiose | 50 - 70 |
| Raffinose | 11 - 30 |
| Stachyose | 6 - 20 |

| | |
|---|---|
| *Activity unit = the amount of enzyme, which is able to hydrolyze 1 µmol PNPG/min in 30°C and in pH 4.5. | |

The α-galactosidase encoded by MEL1 can hydrolyze 0.1 mg stachyose/h/U_{PNPG} in 10 % soya meal (T = 40°C, pH 5.5, 10 U_{PNPG}/g soya meal).

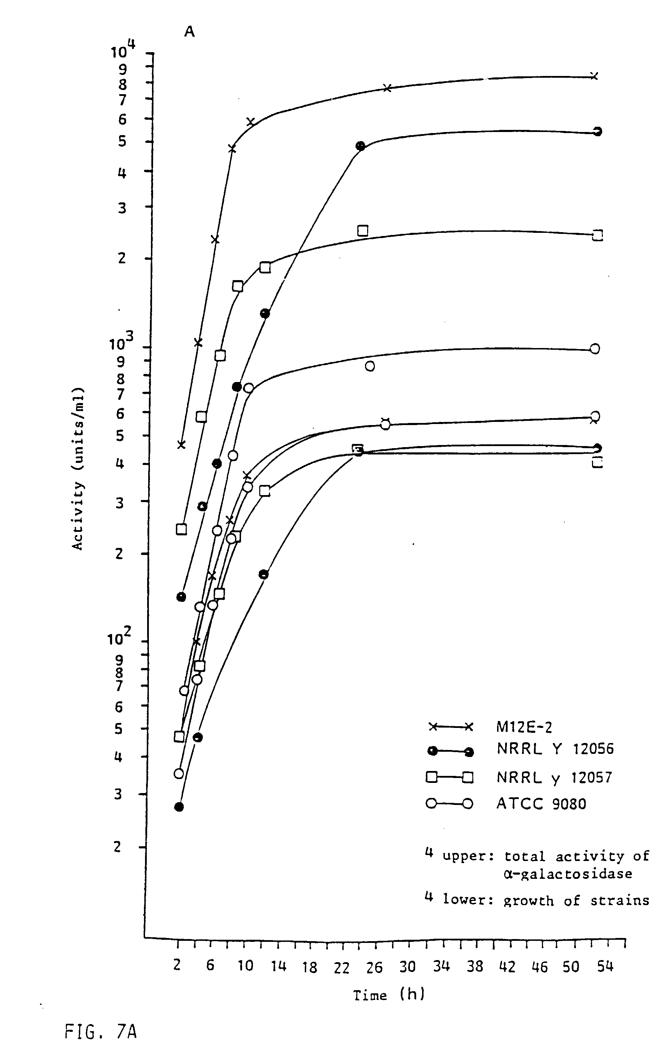

Figure 7A illustrates the kinetics of α-galactosidase production and the total amounts produced during growth in YP+ga15%+glys3%+EtOH2% of different strains. The highest amount of enzyme is produced by M12E-2 (Mel⁺ derivative of baker's yeast). In this case with use of recombinant DNA techniques an 8-fold increase in enzyme production has been achieved. The original strain ATCC9080 produces only 10³U of α-galactosidase when M12E-2 produces 8x10³U.

The higher amount of enzyme produced by M12E-2 is likely to be due to higher copynumber of the MEL1 gene in this strain. In Southern blot (Fig. 2) the new LEU2-hybridizing band is darker compared to the original ones indicating that copynumber of the integrated plasmid is higher than that of the LEU2 gene. This is also supported by the large size of the new band (≥30kb). If only one copy of pALK12 had integrated at LEU2 locus the expected size of the new band would be 16-19 kb.

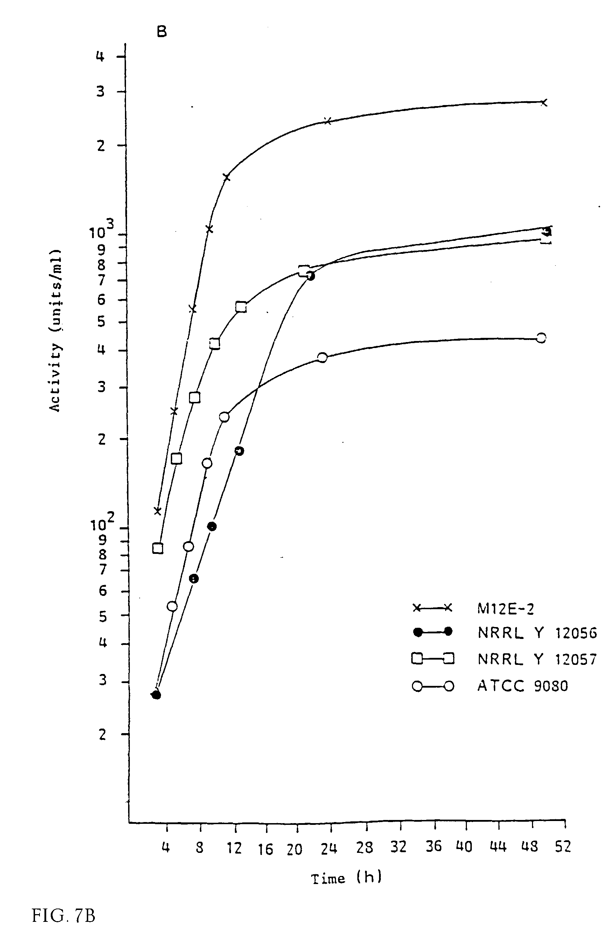

S. cerevisiae var. oleaceus, NRRL Y 12056 (US Patent 4376167) produces almost as high amount of enzyme as M12E-2 but much more slowly. M12E-2 produces 5x10³U of α-galactosidase in 8h when for NRRL Y 12056 it takes 24h (Fig. 7A). A second advantage of M12E-2 when compared to NRRL Y 12056 and NRRL Y 12057 (US Patent 4431737) is that it secretes 3 times more α-galactosidase in culture medium (Fig. 7B). This is important if the enzyme is to be purified from the spent medium, as might be desirable in for example, producing α-galactosidase as a by-product of baker's yeast manufacture. Addition of the strain NRRL Y 12057 (US 4431 737) to soya bean meal has been proposed. This yeast contained 1.2 · 10⁵ units of α-galactosidase activity (equals 11000 U used in US 4431 737) per gram of dry microbial cells. When M12E-2 is grown in fermentor as described in Example 4, it contained 7.8 · 10⁵ U per gram of yeast dry matter.

As mentioned earlier for applications in sugar industry preparations of α-galactosidase should be invertase free. For this purpose the MEL1 gene can be transferred into a Suc⁻ mutant of S. cerevisiae to increase α-galactosidase production. An available S. cerevisiae Mel⁺ Suc⁻ mutant 1453-3A (Suc MAL2 MEL1 his4 leu2, Yeast Genetic Stock Center, Berkeley, California) when transformed with the MEL1 gene produces already about the same amount of enzyme as the naturally occurring Suc⁻ Mel⁺ yeasts NRRL Y 12056 and NRRL Y 12057 (Table 3). The MEL1 gene could be transferred into 1453-3A using pALK2 or pALK12 and the LEU2 gene as a selectable marker since 1453-3A is a leu2⁻ mutant. This should increase the amount of α-galactosidase produced by 1453-3A, since it has already been demonstrated above that increase in copynumber of the MEL1 gene seems to increase the enzyme production as well: compare M12E-2 to M20E-1 and ATCC9080 in Table 3. Alternatively, high level production of α-galactosidase by a Suc⁻ strain could be achieved by inactivating the SUC gene(s) in a Mel⁺ strain such as M12E-2.

The yeast itself containing α-galactosidase activity in the cell wall could be used as a feed supplement as proposed above. This is attractive particularly for the utilization of surplus brewer's yeast. As is shown in Table 3 most (over 70 %) of the α-galactosidase produced by the Mel⁺ transformants of baker's yeast (i.e. strains M12E-2 and M20E-1) was cell bound. In the lager strain NCYC 1324 (naturally Mel⁺) all α-galactosidase activity was cell bound, but the amount of enzyme present was very low. In the Mel⁺ derivative of an ale strain (N12E-1) the amount of cell bound enzyme (55 % of total) is more than 10 fold that naturally present in NCYC 1324.

### EXAMPLE 9

### Increase of α-galactosidase production

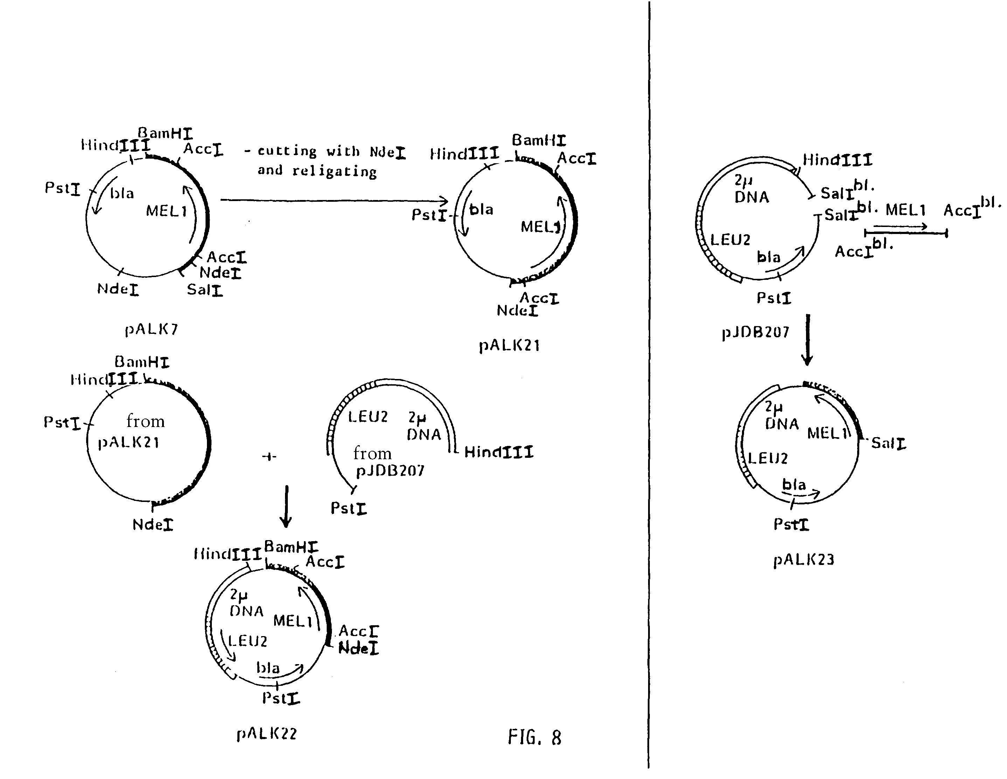

Using recombinant DNA techniques for production of α-galactosidase producing strains can be further developed. Here we demonstrate that by deleting parts of the 5'regulatory region of the MEL1 gene the level of α-galactosidase expression in glucose medium is increased (Table 5). The wild type MEL1 gene is not transcribed in the presence of glucose (Post-Beittenmiller, M., Hamilton, R. and Hopper, J. (1984) Mol. Cell. Biol. 4: 1238-1245). All the strains in Table 5 carry the MEL1 gene on a multicopy plasmid pJDB207 (Beggs, J. (1981) In: Williamson, R. (ed.) Genetic engineering 2. Academic Press, New York pp. 175-203). The plasmid pALK4 contains about 2.8 kb of the MEL1 5' region (Ruohola, H., Liljeström, P., Torkkeli, T., Kopu, H., Lehtinen, P., Kalkkinen, N. and Korhola, M. (1986) FEMS Microbiology Letters 34: 179-185), which is assumed to contain all regulatory sequences. The α-galactosidase activity (31 U, Table 5) in glucose media is probably due to the high copynumber of the MEL1 gene leading to partial escape of normal glucose repression. The other two plasmids pALK22 and pALK23 contain only 243 bp and 175bp of the MEL1 5' region, respectively. The construction of these plasmids has been done in the following way (Fig. 8). The plasmid pALK7 was cut with NdeI and religated resulting in deletion of the smaller NdeI fragment (plasmid pALK21). The larger HindIII-PstI fragment of this plasmid was then ligated with HindIII-PstI-cut pJDB207 to provide selectable marker (LEU2) and replication origin in yeast. The resulting plasmid was called pALK22. To construct a plasmid with even shorter MEL1 5' region the MEL1 gene containing AccI-AccI fragment from pALK7 was isolated and the sticky ends were filled in. This fragment was then ligated with SalI-cut pJDB207, where the sticky ends had also been filled in. The resulting plasmid was called pALK23.

As shown in Table 5 normal regulation of MEL1 expression in strains carrying these plasmids is abolished. Quite high expression occurs in the presence of glucose (148 U and 441 U); in pALK23/YF135 even 14 fold higher than in pALK4/YF135. Since the vector part of these plasmids is the same there shouldn't be any significant difference in the copynumber of the MEL1 gene and the higher expression of the MEL1 gene on plasmid pALK23 is likely to be due to the altered 5' region.

To increase MEL1 expression further high efficiency promoters (e.g. PGK or ADH; M.J. Dobson, Tuite, M.E., Roberts, N.A., Kingsman,A.J., Kingsman, S., Perkins,R.E., Conroy,S.C., Dunbar,B. & Fothergill,L.A. (1982) Nucl.Acids Res. 10: 2625-2637; Ammerer,G. In: Methods in Enzymology 101. Academic Press, New York, pp. 192-201) could be used to direct MEL1 transcription.

**Table 5.**

| Total α-galactosidase activities in 5' deletion strains | | |
|---|---|---|
| Strain | glc 5 % | Carbon source gal 5 % + glyc 3% + EtOH 2 % |
| pALK4/YF135* | 31 | 1146 |
| pALK22/YF135 | 148 | 688 |
| pALK23/YF135 | 441 | 866 |

| | | |
|---|---|---|
| *YF135, α, leu 2-3, 112, his 3-11, 15 (Yeast strain YF135 has been obtained from recombinant DNA Methodology Course in Ottawa in August 1982) | | |

### METHODS

### Measurement of α-galactosidase levels

α-galactosidase levels were measured essentially as described by Kew and Douglas (Kew, O. and Douglas, H. (1976) J. Bacteriol. 125: 33-41). Yeast cells were grown in appropriate medium and samples were taken and chilled on ice. When total activities were measured the culture as such was used as sample. The final reaction mixture (450 µl) contained 50 mM PNPGal (p-nitrophenyl-α-D-galactopyranoside) buffered to pH 4.0 with 31 mM citric acid and 39 mM KH₂PO₄. Reactions were carried out at +30°C and were terminated by addition of 4.5 ml of 0.1 M Na₂CO₃, pH 11.2. the cells were then removed by centrifugation and the p-nitrophenol in the supernatant fluid was measured at 410 nm. One unit of enzyme activity is defined as the amount which hydrolyzes 1.0 nmol of substrate per minute under the conditions described above.

### Culture media

The minimal media contained 0.67 % of YNB medium (Difco) and 2 % of sugar solidified with 2 % agar. When regenerating protoplasts 1M sorbitol was added. As a rich basic medium was used YP medium (1 % Bacto yeast -extract, 2 % bacto peptone). Strains, able or unable to ferment melibiose, were separated with fermentation indicator medium. Because of the pH change the melibiose fermenting strains will change the indicator yellow. This media consisted of 1 % Bacto-yeast extract, 2 % Bacto-peptone, 2 % melibiose, 2 % Bacto-agar and 8 ml/l of 0.4 % brom cresol purple solution. X-α-agal was used on plates in a following way: X-α-gal was dissolved in N'N'-dimethylformamide (20 mg/ml) and 0.1 ml of this solution was spread on agar plates (e.g. YPD, YP + galactose, YNB + melibiose).

The above mentioned plasmid and yeast strain constructions has been nade by using the MEL1 gene of Saccharomyces cerevisiae var. uvarum. It will be understood that the use of the Saccharomyces MEL1 gene is by way of example only. Use of the MEL1 gene as a DNA probe has shown that a family of MEL genes exists within the genus Saccharomyces. The other MEL genes can be differentiated on the basis of differences in DNA sequence, as revealed by restriction enzyme digestion and Southern hybridization (Tubb, R., Liljeström, P., Torkkeli, T. & Korhola, M. (1986) 13th International Conference on Yeast Genetics and Molecular Biology, Banff, Alberta, Canada 1986, Yeast 2 (Spec. Iss.) 1986 S396). Therefore it is feasible to consider use of a MEL gene cloned from other Saccharomyces strains, from other yeasts or from any organism able to produce a suitable α-galactosidase. The means to obtain expression of heterologous genes in yeast will be evident to those skilled in the art of recombinant DNA technology.

## Claims

1. A method for the production of recombinant baker's, distiller's or brewer's yeast strains of the species Saccharomyces cerevisiae, stably comprising a gene coding for an α-galactosidase enzyme and producing increased amounts of said α-galactosidase enzyme compared to the non-transformed starting strain; said method comprising the steps of:
(a) isolating a gene coding for an α-galactosidase enzyme from a yeast strain naturally containing a gene encoding this enzyme;
(b) linking the gene operatively to a vector able to transform and to be maintained in baker's, distiller's or brewer's yeast cells;
(c) transferring this vector or part of it to an industrial baker's, distiller's or brewer's yeast cell; and
(d) directly selecting stable yeast clones expressing said α-galactosidase as a dominant marker and making pure cultures of these, said dominant marker integrated into a chromosome being detected by using:
(i) melibiose, or
(ii) X-α-gal (5-bromo-4-chloro-3-indolyl-α-D-galactoside) as a substrate in the selection and/or screening medium, or
(iii) by using melibiose as the sole carbon source and X-α-gal as a substrate in the selection and/or screening medium, or
selecting stable yeast clones expressing said α-galactosidase as a dominant marker and making pure cultures of these, said dominant marker contained in an autonomously replicating plasmid being detected by using:
(i) X-α-gal as a substrate in the selection and/or screening medium, or
(ii) by using melibiose as the sole carbon source and X-α-gal as a substrate in the selection and/or screening medium.

2. The method according to claim 1 , wherein the gene is the MEL1 gene from Saccharomyces cerevisiae var. uvarum.

3. The method according to claim 1 or 2, wherein the plasmid containing a gene coding for an α-galactosidase enzyme comprises at least part of plasmids pALK2; pALK4; pALK12 (having the restriction map shown in Figure 1); pALK20 (having the restriction map shown in Figure 3); pALK22 (having the restriction map evident from Figure 1 and Figure 8); pALK23 (having the restriction map shown in Figure 8); or pALK52 (having the restriction map shown in Figure 4) or any combination of these plasmids.

4. The method according to any one of claims 1 to 3, wherein the transformed yeast strain does not produce invertase.

5. The method according to any one of claims 1 to 3, wherein the transformed yeast strain is MK270.

6. The method according to any one of claims 1 to 3, wherein the transformed yeast strain in NCYC 1245.

7. The method according to any one of claims 1 to 3, wherein the transformed yeast strain is YF135.

8. The method according to any one of claims 1 to 3 or 5 to 7, wherein the yeast strain is M12E-2 (NCYC 1567); M20E-1 (NCYC 1568); N12E-1 (NCYC 1569); pALK22/YF135, obtained by transforming strain YF135 with plasmid pALK22, the restriction map of which is shown in Figure 8; pALK23/YF135, obtained by transforming strain YF135 with plasmid pALK23, the restriction map of which is shown in Figure 8; pALK4/MK270, obtained by transforming strain MK270 with plasmid pALK4; or pALK52/MK270, obtained by transforming strain MK270 with plasmid pALK52, the restriction map of which is shown in Figure 4.

9. Baker's, distiller's or brewer's yeast strain of the species Saccharomyces cerevisiae, characterised in that it is an industrially applicable transformant of a wild-type strain stably comprising a gene coding for an α-galactosidase enzyme, said yeast strain being able to produce increased amounts of said α-galactosidase enzyme compared to the non-transformed starting strain, and obtainable according to the method of any one of claims 1 to 8.

10. Use of a plasmid, wherein said plasmid is able to stably transform and express a gene coding for an α-galactosidase enzyme in an industrially used baker's, distiller's or brewer's yeast strain, wherein said gene is the MEL1 gene from Saccharomyces cerevisiae var. uvarum in the method of any one of claims 1 to 8.

11. The use according to claim 10, wherein said plasmid comprises at least part of the plasmids pALK2; pALK4; pALK12 (having the restriction map shown in Figure 1); pALK20 (having the restriction map shown in Figure 3); pALK22 (having the restriction map evident from Figure 1 and Figure 8); pALK23 (having the restriction map shown in Figure 8); or pALK52 (having the restriction map shown in Figure 4) or any combination of these plasmids.

12. Use of the baker's or distiller's yeast strain according to claim 9 for the utilisation of raffinose or higher α-galactosides from a raw material.

13. Use of the baker's or distiller's yeast strain according to claim 9 for the production of an α-galactosidase enzyme in a method which comprises:
(a) cultivating said yeast strain under suitable conditions; and
(b) recovering and purifying the α-galactosidase enzyme.

14. Use of a brewer's yeast strain according to claim 9 for detecting the pasteurisation units of beer in a method which comprises:
(a) preparing beer using the α-galactosidase-producing brewer's yeast strain, the α-galactosidase production of which is enhanced compared to the α-galactosidase production of naturally occurring brewer's yeast strains which are able to produce this enzyme;
(b) pasteurising the beer produced; and
(c) measuring the α-galactosidase activity of the beer.

15. Use of a brewer's yeast strain according to claim 9 in a method according to claim 14, wherein the α-galactosidase activity of the beer is measured by using X-α-gal (5-bromo-4-chloro-3-indolyl-α-D-galactoside) as a chromogenic substrate.

16. Use of a gene encoding an α-galactosidase enzyme as a dominant marker for marking an industrially applicable yeast strain whereby said gene is transferred to and integrated into the chromosome of said yeast strain and subsequently detected by conventional hybridisation methods.

17. Use according to claim 16, wherein said gene is the MEL1 gene from the species Saccharomyces cerevisiae var. uvarum.

## Patentansprüche

1. Verfahren zur Produktion von rekombinanten Back-, Brennerei- oder Bierhefe-Stämmen der Art Saccharomyces cerevisiae, die in stabiler Form ein ein α-Galactosidaseenzym codierendes Gen umfassen und erhöhte Mengen des α-Galactosidaseenzyms produzieren, verglichen mit dem nicht transformierten Ausgangsstamm, wobei das Verfahren folgende Schritte umfaßt:
(a) Isolieren eines ein α-Galactosidaseenzym codierenden Gen aus einem Hefestamm, der natürlicherweise ein dieses Enzym codierendes Gen enthält,
(b) funktionelle Verknüpfung des Gens mit einem Vektor, der Back-, Brennerei- oder Bierhefezellen transformieren kann und darin gehalten werden kann,
(c) Einbringen des Vektors oder eines Teils davon in eine industrielle Back-, Brennerei- oder Bierhefezelle, und
(d) direkte Selektion stabiler Hefeclone, die die α-Galactosidase als dominanten Marker exprimieren, und Herstellung von Reinkulturen davon, wobei der in ein Chromosom integrierte dominante Marker folgendermaßen nachgewiesen wird:
(i) Verwendung von Melibiose oder
(ii) X-α-gal (5-Brom-4-chlor-3-indolyl-α-D-galactosid) als Substrat in dem Selektions- und/oder Durchmusterungsmedium, oder
(iii) Verwendung von Melibiose als einziger Kohlenstoffquelle und von X-α-gal als Substrat in dem Selektions- und/oder Durchmusterungsmedium, oder
Selektion stabiler Hefeclone, die α-Galactosidase als einen dominanten Marker exprimieren, und Herstellung von Reinkulturen davon, wobei der in einem autonom replizierbaren Plasmid enthaltene dominante Marker folgendermaßen nachgewiesen wird:
(i) Verwendung von X-α-gal als Substrat im Selektions- und/oder Durchmusterungsmedium, oder
(ii) Verwendung von Melibiose als einziger Kohlenstoffquelle und X-α-gal als Substrat im Selektions- und/oder Durchmusterungsmedium.

2. Verfahren nach Anspruch 1, wobei das Gen das MEL1-Gen von Saccharomyces cerevisiae var. uvarum ist.

3. Verfahren nach Anspruch 1 oder 2, wobei das Plasmid, das ein ein α-Galactosidaseenzym codierendes Gen enthält, mindestens einen Teil der Plasmide pALK2, pALK4, pALK12 (mit der in Figur 1 gezeigten Restriktionskarte), pALK20 (mit der in Figur 3 gezeigten Restriktionskarte), pALK22 (mit der aus den Figuren 1 und 8 ersichtlichen Restriktionskarte), pALK23 (mit der in Figur 8 gezeigten Restriktionskarte) oder pALK52 (mit der in Figur 4 gezeigten Restriktionskarte) oder beliebige Kombinationen dieser Plasmide umfaßt.

4. Verfahren nach einem der Ansprüche 1 bis 3, wobei der transformierte Hefestamm keine Invertase produziert.

5. Verfahren nach einem der Ansprüche 1 bis 3, wobei der transformierte Hefestamm MK270 ist.

6. Verfahren nach einem der Ansprüche 1 bis 3, wobei der transformierte Hefestamm NCYC 1245 ist.

7. Verfahren nach einem der Ansprüche 1 bis 3, wobei der transformierte Hefestamm YF135 ist.

8. Verfahren nach einem der Ansprüche 1 bis 3 oder 5 bis 7, wobei folgende Hefestämme erhalten werden: M12E-2 (NCYC 1567), M20E-1 (NCYC 1568); N12E-1 (NCYC 1569); pALK22/YF135, erhalten durch Transformation von Stamm YF135 mit Plasmid pALK22, dessen Restriktionskarte in Figur 8 gezeigt ist, pALK23/YF135, erhalten durch Transformation vcm Stamm YF135 mit Plasmid pALK23, dessen Restriktionskarte in Figur 8 gezeigt ist, pALK4/MK270, erhalten durch Transformation vom Stamm MK270 mit Plasmid pALK4, oder pALK52/MK270, erhalten durch Transformation von Stamm MK270 mit Plasmid pALK52, dessen Restriktionskarte in Figur 4 gezeigt ist.

9. Back-, Brennerei- oder Bierhefestamm der Art Saccharomyces cerevisiae, dadurch gekennzeichnet, daß es eine industriell verwendbare Transformante eines Wildtypstamms ist, umfassend in stabiler Form ein ein α-Galactosidaseenzym codierendes Gen, wobei der Hefestamm erhöhte Mengen des α-Galactosidaseenzyms produzieren kann, verglichen mit dem nicht-transformierten Ausgangsstamm, und erhältlich gemäß dem Verfahren nach einem der Ansprüche 1 bis 8.

10. Verwendung eines Plasmids, wobei das Plasmid ein ein α-Galactosidaseenzym codierendes Gen in stabiler Form in einen industriell verwendeten Back-, Brennerei- oder Bierhefestamm transformieren und exprimieren kann, wobei das Gen das MEL1-Gen von Saccharomyces cerevisiae var. uvarum ist, im Verfahren nach einem der Ansprüche 1 bis 8.

11. Verwendung nach Anspruch 10, wobei das Plasmid mindestens einen Teil der Plasmide pALK2, pALK4, pALK12 (mit der in Figur 1 gezeigten Restriktionskarte), pALK20 (mit der in Figur 3 gezeigten Restriktionskarte), pALK22 (mit der aus Figur 1 und Figur 8 ersichtlichen Restriktionskarte), pALK23 (mit der in Figur 8 gezeigten Restriktionskarte) oder pALK52 (mit der in Figur 4 gezeigten Restriktionskarte) oder eine beliebige Kombination dieser Plasmide umfaßt.

12. Verwendung des Back- oder Brennereihefestamms nach Anspruch 9 für die Verwertung von Raffinose oder höheren α-Galactosiden aus einem Rohmaterial.

13. Verwendung des Back- oder Brennereihefestamms nach Anspruch 9 für die Produktion eines α-Galactosidaseenzyms in einem Verfahren mit folgenden Schritten:
(a) Züchtung des Hefestamms unter geeigneten Bedingungen und
(b) Gewinnung und Reinigung des α-Galactosidaseenzyms.

14. Verwendung eines Bierhefestamms nach Anspruch 9 zum Nachweis der Pasteurisierungseinheiten von Bier in einem Verfahren mit folgenden Schritten:
(a) Herstellung von Bier unter Verwendung des α-Galactosidase-produzierenden Bierhefestamms, dessen α-Galactosidaseproduktion erhöht ist, verglichen mit der α-Galactosidaseproduktion von natürlich vorkommenden Bierhefestämmen, die dieses Enzym produzieren können,
(b) Pasteurisieren des produzierten Bieres, und
(c) Messen der α-Galactosidaseaktivität des Bieres.

15. Verwendung eines Bierhefestammes nach Anspruch 9 in einem Verfahren gemäß Anspruch 14, wobei die α-Galactosidaseaktivität des Bieres unter Verwendung von X-α-gal (5-Brom-4-chlor-3-indolyl-α-D-galactosid) als chromogenem Substrat gemessen wird.

16. Verwendung eines ein α-Galactosidasenzym codierenden Gens als dominanten Marker zur Markierung eines industriell verwendbaren Hefestammes, wobei das Gen in das Chromosom des Hefestammes eingebracht und integriert wird und anschließend durch übliche Hybridisierungsverfahren nachgewiesen wird.

17. Verwendung nach Anspruch 16, wobei das Gen das MEL1-Gen aus der Art Saccharomyces cerevisiae var. uvarum ist.

## Revendications

1. Procédé de production de souches recombinées de levure de boulanger, de distillation ou de bière de l'espèce Saccharomyces cerevisiae, comprenant de façon stable un gène codant pour une enzyme α-galactosidase et produisant des quantités accrues de ladite enzyme α-galactosidase par rapport à la souche de départ non transformée ; ledit procédé comprenant les étapes consistant à :
(a) isoler un gène codant pour une enzyme α-galactosidase à partir d'une souche de levure contenant naturellement un gène codant pour cette enzyme ;
(b) lier le gène de façon opératoire à un vecteur capable de se transformer et d'être maintenu dans des cellules de levure de boulanger, de distillation ou de bière ;
(c) transférer ce vecteur ou une partie de celui-ci dans une cellule de levure industrielle de boulanger, de distillation ou de bière ; et
(d) choisir directement les clones de levure stables exprimant ladite α-galactosidase en tant que marqueur dominant et réaliser des cultures pures de ces clones, ledit marqueur dominant intégré dans un chromosome étant détecté en utilisant :
(i) du mélibiose ou
(ii) du X-α-gal (5-bromo-4-chloro-3-indolyl-α-D-galastoside) en tant que substrat dans le milieu de sélection et/ou de criblage ou
(iii) du mélibiose en tant que seule source carbonée et du X-α-gal en tant que substrat dans le milieu de sélection et/ou de criblage, ou choisir les clones de levure stables exprimant ladite α-galactosidase en tant que marqueur dominant et réaliser des cultures pures de ces clones, ledit marqueur dominant contenu dans un plasmide à réplication autonome étant détecté en utilisant :
(i) du X-α-gal en tant que substrat dans le milieu de sélection et/ou de criblage ou
(ii) du mélibiose en tant que seule source carbonée et du X-α-gal en tant que substrat dans le milieu de sélection et/ou de criblage.

2. Procédé selon la revendication 1, dans lequel le gène est le gène MEL1 provenant de Saccharomyces cerevisiae var. uvarum.

3. Procédé selon la revendication 1 ou 2, dans lequel le plasmide contenant un gène codant pour une enzyme α-galactosidase comprend au moins une partie des plasmides pALK2, pALK4, pALK12 (ayant la carte de restriction représentée sur la figure 1), pALK20 (ayant la carte de restriction représentée sur la figure 3), pALK22 (ayant la carte de restriction évidente de la figure 1 et de la figure 8), pALK23 (ayant la carte de restriction représentée sur la figure 8) ou pALK52 (ayant la carte de restriction représentée sur la figure 4) ou l'une quelconque des associations de ces plasmides.

4. Procédé selon l'une quelconque des revendications 1 à 3, dans lequel la souche de levure transformée ne produit pas l'invertase.

5. Procédé selon l'une quelconque des revendications 1 à 3, dans lequel la souche de levure transformée est MK270.

6. Procédé selon l'une quelconque des revendications 1 à 3, dans lequel la souche de levure transformée est NCYC 1245.

7. Procédé selon l'une quelconque des revendications 1 à 3, dans lequel la souche de levure transformée est YF135.

8. Procédé selon l'une quelconque des revendications 1 à 3 ou 5 à 7, dans lequel la souche de levure est M12E-2 (NCYC 1567), M20E-1 (NCYC 1568), N12E-1 (NCYC 1569), pALK22/YF135 obtenu par transformation de la souche YF135 avec le plasmide pALK22 dont la carte de restriction est représentée sur la figure 8, pALK23/YF135 obtenu par transformation de la souche YF135 avec le plasmide pALK23 dont la carte de restriction est représentée sur la figure 8, pALK4/MK270 obtenu par transformation de la souche MK270 avec le plasmide pALK4, ou bien pALK52/MK270 obtenu par transformation de la souche MK270 avec le plasmide pALK52 dont la carte de restriction est représentée sur la figure 4.

9. Souche de levure de boulanger, de distillation ou de bière de l'espèce Saccharomyces cerevisiae, caractérisée en ce que c'est un transformant applicable dans l'industrie d'une souche de type sauvage comprenant de façon stable un gène codant pour une enzyme α-galactosidase, ladite souche de levure étant capable de produire des quantités accrues de ladite enzyme α-galactosidase par rapport à la souche de départ non transformée et pouvant être obtenue selon le procédé de l'une quelconque des revendications 1 à 8.

10. Utilisation d'un plasmide, dans laquelle ledit plasmide est capable de se transformer de façon stable et d'exprimer un gène codant pour une enzyme α-galactosidase dans une souche de levure de boulanger, de distillation ou de bière utilisée dans l'industrie, dans laquelle ledit gène est le gène MEL1 provenant de Saccharomyces cerevisiae var. uvarum, dans le procédé de l'une quelconque des revendications 1 à 8.

11. Utilisation selon la revendication 10, dans laquelle ledit plasmide comprend au moins une partie des plasmides pALK2, pALK4, pALK12 (ayant la carte de restriction représentée sur la figure 1), pALK20 (ayant la carte de restriction représentée sur la figure 3), pALK22 (ayant la carte de restriction évidente de la figure 1 et de la figure 8), pALK23 (ayant la carte de restriction représentée sur la figure 8) ou pALK52 (ayant la carte de restriction représentée sur la figure 4) ou l'une quelconque des associations de ces plasmides.

12. Utilisation de la souche de levure de boulanger ou de distillation selon la revendication 9 pour l'utilisation du raffinose ou d'α-galactosides supérieurs provenant d'un produit brut.

13. Utilisation de la souche de levure de boulanger ou de distillation selon la revendication 9 pour la production d'une enzyme α-galactosidase dans un procédé qui comprend:
(a) la culture de ladite souche de levure dans les conditions adaptées ; et
(b) la récupération et la purification de l'enzyme α-galactosidase.

14. Utilisation d'une souche de levure de bière selon la revendication 9, pour la détection des unités de pasteurisation de la bière dans un procédé qui comprend:
(a) la préparation de bière en utilisant la souche de levure de bière productrice d'α-galactosidase dont la production d'α-galactosidase est accrue par rapport à la production d'α-galactosidase des souches de levure de bière d'origine naturelle qui sont capables de produire cette enzyme ;
(b) la pasteurisation de la bière produite ; et
(c) la mesure de l'activité d'α-galactosidase de la bière.

15. Utilisation d'une souche de levure de bière selon la revendication 9 dans un procédé selon la revendication 14, dans laquelle l'activité d'α-galactosidase de la bière est mesurée en utilisant du X-α-gal (5-bromo-4-chloro-3-indolyl-α-D-galactoside) en tant que substrat chromogène.

16. Utilisation d'un gène codant pour une enzyme α-galactosidase en tant que marqueur dominant destiné au marquage d'une souche de levure applicable dans l'industrie, ledit gène étant de ce fait transféré et intégré dans le chromosome de ladite souche de levure et ensuite détecté par des procédés classiques d'hybridation.

17. Utilisation selon la revendicatioin 16, dans laquelle ledit gène est le gène MEL1 provenant de l'espèce Saccharomyces cerevisiae var. uvarum.
